(19) Europäisches Patentamt
European Patent Office
Office européen des brevets

(11) EP 2 237 033 B1

(12) EUROPEAN PATENT SPECIFICATION

(45) Date of publication and mention of the grant of the patent:
**27.06.2012 Bulletin 2012/26**

(51) Int Cl.:
***G01N 33/493*** *(2006.01)* ***G01N 15/14*** *(2006.01)*

(21) Application number: **10158140.3**

(22) Date of filing: **29.03.2010**

(54) **Urine sample analyzer**

Analysegerät für Urinproben

Analyseur d'échantillon d'urine

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **30.03.2009 JP 2009081852**

(43) Date of publication of application:
**06.10.2010 Bulletin 2010/40**

(73) Proprietor: **SYSMEX CORPORATION**
**Kobe-shi,**
**Hyogo 651-0073 (JP)**

(72) Inventors:
- **Tsutsumida, Keisuke**
  **Hyogo 651-0073 (JP)**
- **Tanaka, Yousuke**
  **Hyogo 651-0073 (JP)**
- **Minato, Yukio**
  **Hyogo 651-0073 (JP)**
- **Mizumoto, Toru**
  **Hyogo 651-0073 (JP)**

(74) Representative: **HOFFMANN EITLE**
**Patent- und Rechtsanwälte**
**Arabellastraße 4**
**81925 München (DE)**

(56) References cited:
**EP-A2- 1 638 029**
**EP-A2- 1 857 804**
**JP-A- 11 064 218**
**US-A1- 2006 073 606**
**US-A1- 2007 072 301**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

FIELD OF THE INVENTION

The present invention relates to a urine analyzer.

BACKGROUND

Conventional urine sample analyzers for analyzing urine samples are disclosed in, for example, United States Patent No. 5,325,168 and Japanese Patent Publication No. 2001/228158.

United States Patent No. 5,325,168 discloses a cell analyzer for classifying and counting cells such as leukocytes, erythrocytes, epithelial cells, urinary casts, bacteria and the like found in urine.

Japanese Patent Publication No. 2001/228158 discloses an automatic analyzer for quantifying the protein components and lipid components in urine.

In diagnosing renal disease, a urine analysis (urine sediment analysis) by cell analyzers such as that disclosed in United States Patent No. 5,325,168 and a urine analysis (urine biochemical analysis) by automatic analyzers such as that disclosed in Japanese Patent Publication No. 2001/228158 are conventionally performed at different stages. Specifically, urine analysis (urine sediment analysis) is generally performed repeatedly using a cell analyzer such as that disclosed in United States Patent No. 5,325, 168 from the early to intermediate stage of analysis, whereas urine analysis (urine biochemical analysis) is typically performed by cell analyzers such as that disclosed in Japanese Patent Publication No. 2001/228158 at the late stage of analysis.

Further, EP 1 857 804 A2 relates to an apparatus for analyzing particles in urine and shows a first apparatus for measuring particles in urine containing red blood cells and a second apparatus for measuring bacteria.

In addition, JP 11 064218 A, relates to a method for quantifying a concentration of body fluid component and considers light absorption in the near-infrared and thus the measurement of CH radical-oriented absorption, OH radical-oriented absorption, and NH radical-oriented absorption.

Since the conventional process of diagnosing renal disease involves repeated urine sediment analyses at the early and intermediate stages of analysis, and then performing urine biochemical analysis at the late stage, urine sedimentation analysis results and urine biochemical analysis results can not both be obtained at the early stage. As a result, a problem arises in that useful analysis results are unavailable at an early stage for renal disease diagnosis.

SUMMARY

The present invention is a urine analyzer according to claim 1 comprising: a first measurement unit for obtaining red blood cell measurement information by performing a measurement of a red blood cell in a urine sample; a second measurement unit for obtaining quantitative measurement information by performing a quantitative measurement of at least one of a protein component and a lipid component in the urine sample; and a data analysis unit for obtaining at least one of numerical information representing the number of red blood cells in the urine sample and morphology information of a red blood cell in the urine sample by analyzing the red blood cell measurement information obtained by the first measurement unit, and for obtaining concentration information representing a concentration of at least one of the protein component and the lipid component in the urine sample by analyzing the quantitative measurement information obtained by the second measurement unit.

According to this configuration, at least one of the numerical information representing the red blood cell count in the urine sample and the morphological information indicating the morphology of the red blood cells in the urine sample are obtained as the urine sediment analysis result, and the concentration information indicating the concentration of at least one of the protein component and lipid component in the urine sample is obtained as the urine biochemical analysis result in the same analysis stage. Thus, both urine sediment analysis results and urine biochemical analysis results can be obtained at an early stage, and as a result analysis results useful for diagnosis of renal disease can be obtained at an early stage compared to when urine biochemical analysis is performed after repeatedly performing urine sediment analyses. Since the red blood cell measurement information obtained by the first measurement unit and the quantitative measurement information obtained by the second measurement unit are both analyzed by a single data analysis unit, providing separate analysis units for the first measurement unit and the second measurement unit is unnecessary. As a result, the overall structure of the analyzer can be simplified while providing a plurality of measurement units.

The urine sample analyzer according to the invention further comprises an operation controller shared to control the first measurement unit and the second measurement unit.

According to this configuration, it is unnecessary to provide separate controllers for the first measurement unit and the second measurement unit since the operations of the first measurement unit and the second measurement unit are controlled by the joint controller. As a result, the overall structure of the analyzer can be simplified while providing a plurality of measurement units.

Preferably, the protein component comprises at least one of total protein and albumin; and the lipid component comprises cholesterol.

According to this configuration, it is possible to obtain concentration information representing the concentration of at least one of total protein component, albumin, and cholesterol in the urine sample as the urine biochemical analysis result. Note that in the present specification, the meaning of total protein albumin, and cholesterol broadly includes micro total protein, micro albumin, and micro cholesterol.

Preferably, the data analysis unit comprises: a display; and a controller for controlling the display so as to display, on a same screen, the concentration information and at least one of the numerical information and morphological information.

According to this configuration, the user (physician) can easily confirm the concentration information and at least one of the numerical information and morphological information visually on the same screen.

Preferably, the controller obtains diagnostic support information relating at least to renal disease based on the concentration information and at least one of the numerical information and morphological information, and controls the display so as to display, on the screen, the obtained diagnostic support information.

Preferably, the data analysis unit outputs diagnostic support information relating at least to renal disease based on the concentration information and at least one of the numerical information and red blood cell morphological information.

According to this configuration, the user (physician) can use the provided diagnostic information for the diagnosis of renal disease.

The urine sample analyzer according to the invention further comprises a transport unit for transporting a sample container containing the urine sample, the first measurement unit aspirates the urine sample from the sample container transported by the transporting unit, and perform the measurement of the urine sample; and the second measurement unit aspirates the urine sample from the sample container transported by the transporting unit, and perform the measurement of the urine sample.

According to this configuration, the urine sample can be provided easily to the first measurement unit and the second measurement unit by the transporting unit.

The first measurement unit aspirates the urine sample from the sample container transported to a first aspirating position by the transporting unit; the second measurement unit aspirates the urine sample from the sample container transported to a second aspirating position by the transporting unit; and the transporting unit comprises a circulating transport path on which the first aspirating position and the second aspirating position are arranged, and transports the sample container to the first aspirating position and the second aspirating position along the circulating transport path.

According to this configuration, the urine sample can be transported easily to the second aspirating position after being transported to the first aspirating position, and the urine sample can be transported easily to the first aspirating position after being transported to the second aspirating position, Thus, the urine sample can be re-transported easily to the first aspirating position and the second aspirating position when remeasurement is required by the first measurement unit and the second measurement unit.

The data analysis unit receives an instruction to execute the quantitative measurement by the second measurement unit; and the second measurement unit executes the quantitative measurement when the data analysis unit has received the instruction.

According to this configuration, urine sample that do not require quantitative measurement are not subjected to quantitative measurement by the second measurement unit.

The data analysis unit according to the invention determines the necessity of the quantitative measurement by the second measurement unit based on at least one of the numerical information and morphological information; and the second measurement unit executes the quantitative measurement when the data analysis unit has determined that the quantitative measurement by the second measurement unit is necessary.

Preferably, the data analysis unit determines that the quantitative measurement by the second measurement unit is necessary when the number of the red blood cells in the urine sample is higher than a threshold value.

Preferably, the data analysis unit receives an instruction to execute the measurement of red blood cells in the urine sample by the first measurement unit; and the first measurement unit executes the measurement of red blood cells in the urine sample when the data analysis unit has received the instruction to execute the measurement of the red blood cells in the urine sample.

According to this configuration, urine sample that do not require red blood cell measurement are not subjected to measurement by the first measurement unit.

Preferably, the data analysis unit receives a qualitative measurement result relating to the urine sample, and determines the necessity of the measurement of the red blood cells in the urine sample by the first measurement unit based on the received qualitative analysis result relating to the urine sample.

Preferably, the second measurement unit obtains creatinine quantitative measurement information by further performing quantitative measurement of the creatinine in the urine sample; and the data analysis unit corrects the concentration information representing the concentration of at least one of the protein component and lipid component in the urine sample based on the creatinine quantitative measurement information.

According to this configuration, the amount of various components in a urine sample can be accurately obtained without dispersion caused by collection time since an estimated value of the excretion of a component per day is obtained for at least one of the protein component and lipid component in the urine sample. Note that in the present specification, the meaning of creatinine broadly includes micro creatinine.

The first measurement unit and the second measurement unit are disposed within a unitary analyzer body.

*According to this configuration, the urine sample analyzer incorporating the first measurement unit and the second measurement unit occupies a smaller space compared to when the first measurement unit and the second measurement unit are installed separately.

BRIEF DESCRIPTION OF THE DRAWINGS

Fig. 1 is a perspective view showing the general structure of an embodiment of the urine sample analyzer of the present invention;

FIG. 2 is a block diagram showing the urine sample measurement unit of the embodiment of the urine sample analyzer of FIG. 1;

FIG. 3 is a brief illustration of the structure of the urine formed component measurement unit of the embodiment of the urine sample analyzer of FIG. 1;

FIG. 4 is a block diagram showing the data analysis unit of the embodiment of the urine sample analyzer of FIG. 1;

FIG. 5 is a block diagram of the qualitative urine analyzer capable of sending and receiving data to/from the embodiment of the urine sample analyzer of FIG. 1;

FIG. 6 is a flow chart showing the operation of the urine sample analysis process of the embodiment of the urine sample analyzer of FIG. 1;

FIG. 7 is a flow chart showing the operation of the diagnostic support information obtaining process of step S16 shown in FIG. 6; and

FIG. 8 shows an analysis result and diagnostic support screen of the embodiment of the urine sample analyzer of FIG. 1.

DETAILED DESCRIPTION OF THE EMBODIMENT

The embodiments of the present invention is described hereinafter based on the drawings.

The general structure of an embodiment of the urine sample analyzer 1 of the present invention is described below with reference to Figs. 1 through 5.

The urine sample analyzer 1 of the present embodiment is provided with a urine sample measurement unit 2, transporting unit 3, and data analysis unit 4, as shown in FIG. 1. The data analysis unit 4 is configured to be capable of receiving a urine qualitative analysis result from an external urine qualitative analyzer 5.

As shown in FIG. 2, the urine sample measurement unit 2 includes a body 21, a urine formed component measurement unit 22 and biochemical measurement unit 23 disposed within the unitary body 21, a communication unit 24, and controller 25. Both the urine formed component measurement unit 22 and biochemical measurement unit 23 are covered by a unitary housing 211, as shown in FIG. 1.

The urine formed component measurement unit 22 is configured to be capable of obtaining measurement data of each measurement item relating to formed components (red blood cells, white blood cells, epithelial cells, casts, and bacteria) in a supplied urine sample via a flow cytometric method. As shown in FIG. 3, the urine formed component measurement unit 22 further includes, although not shown in the drawings, a reactor, aspirating pipette 221 (refer to FIG. 1 ) for aspirating a urine sample transported by the transporting unit 3, optical detection unit 222, analog signal processing circuit 223, and A/D converter 224 for converting the output of the analog signal processing circuit 223 to digital signals. The urine formed component measurement unit 22 is configured to dilute the urine sample aspirated by the aspirating pipette 221 approximately 4-fold with diluting solution and staining solution in the reactor. The reactor is configured to mix the urine sample, diluting solution, and staining solution in a staining process lasting approximately 10 seconds at a constant temperature of approximately 35°. The urine formed component measurement unit 22 also is configured to supply the urine sample prepared in the reactor, together with a sheath fluid, to a sheath flow cell 222c (refer to FIG. 3; to be described later) of the optical detection unit 222.

As shown in FIG. 3, the optical detection unit 222 includes a light emitter 222a for emitting laser light, illumination lens unit 222b, sheath flow cell 222c which is illuminated by the laser light, a PD (photodiode) 222f and collective lens 222d which are disposed on a line extending in the direction of travel of the laser light emitted from the light emitter 222a, collective lens 222g which is disposed in a direction intersecting the direction of travel of the laser light emitted from the light emitter 222a, dichroic mirror 222h, optical filter 222i, PMT (photomultiplier tube) 222k and (pinhole board 222j provided with a pinhole, And PD 222l which is disposed on the side of the dichroic mirror 222h.

The light emitter 222a is provided to emit light on the sample flow that includes the measurement sample passing through the sheath flow cell 222c. The illumination lens unit 222b is provided to render parallel rays from the light emitted from the light emitter 222a. The PD 222f is provided to receive the forward scattered light emitted from the sheath flow cell 222c.

The dichroic mirror 222h is provided to separate the side scattered light and side fluorescent light emitted from the sheath flow cell 222c. Specifically, the dichroic mirror 222h directs the side scattered light emitted from the sheath flow cell 222c to the PD 2221, and directs the side fluorescent light emitted from the sheath flow cell 222c to the PMT 222k. The PD 2221 is provided to receive the side scattered light. The PMT 222k is provided to receive the side fluorescent light. The PDs 222f and 2221, and the PMT 222k respectively have the function of converting the received optical signals to electrical signals.

The analog signal processing circuit 223 includes amps 223a, 223b, and 223c, as shown in FIG. 3. The amps 223a, 223b, and 223c are respectively provided to perform amplification and waveform processing on the electrical signals output from the PD 222f, PMT 222k, and PD 2221. The amplified and waveform processed electrical signals are output to the controller 25 through the A/D converter 224.

The biochemical measurement unit 23 has the function of obtaining quantitative information relating to the biochemical components contained in the urine sample. Specifically, the biochemical measurement unit 23 is configured to accept the urine sample into the apparatus, and dispense reagents to the accepted urine sample. The biochemical measurement unit 23 also is configured to measure the transmission light produced by the light illuminating the urine sample that contains the dispensed reagent. Thus, the biochemical measurement unit 23 is configured to obtain quantitative information relating to the biochemical components contained in the urine sample by measuring the urine sample using optical methods. Specifically, the biochemical measurement unit 23 is capable of obtaining quantitative biochemical measurement data of the total protein, albumin, cholesterol, and creatinine in the urine sample. The biochemical measurement unit 23 is further configured to aspirate the urine sample transported by the transporting unit 3 via the aspirating pipette 231 (refer to FIG. 1), and accept the sample into the measurement unit.

The communication unit 24 is an Ethernet (registered trademark) interface, and the urine sample measurement unit 2 is connected to the data analysis unit 4 through the communication unit 24 and a LAN cable. The urine sample measurement unit 2 is configured to transmit, to the data analysis unit 4, the various measurement data obtained from the urine formed component measurement unit 22 and the biochemical measurement unit 23 using a predetermined communication protocol (TCP/IP).

The controller 25 has a CPU 25a and a memory 25b. The CPU 25a is configured to control the operations of each unit by executing computer programs stored in the memory 25b. That is, the CPU 25a is configured to control the operations of both the urine formed component measurement unit 22 and the biochemical measurement unit 23.

As shown in FIG. 1, the transporting unit 3 is positioned on the front side (side in the arrow Y1 direction) of the urine sample measurement unit 2. The transporting unit 3 is configured to transport the urine sample to the urine formed component measurement unit 22 and the biochemical measurement unit 23 of the urine sample measuring unit 2. Specifically, the transporting unit 3 is configured to transport a sample container 100 held in a rack 101 to a position (urine sample obtaining position of the urine formed component measurement unit 22) below the aspirating pipette 221 of the urine formed component measurement unit 22 and to a position (urine sample obtaining position of the biochemical measurement unit 23) below the aspirating pipette 231 of the biochemical measurement unit 23 by moving the rack 101.

The transporting unit 3 is also configured to circulate a rack 101 capable of holding a plurality of sample containers 100 so as to pass by the urine sample obtaining position of the urine formed component measurement unit 22 and the urine sample obtaining position of the biochemical measurement unit 23. Specifically, the transporting unit 3 has a first transverse unit 31, second transverse unit 32, and a buffer unit 33 disposed between the first transverse unit 31 and the second transverse unit 32. The transporting unit 3 also has a rack feeder 34 for feeding the rack 101 to the first transverse unit 31, rack take-up 35 for taking up the rack 101 from the second transverse unit 32, and a third transverse unit 36 disposed to the front of the buffer unit 33 and connected to the rack feeder 34 and tack take-up 35. That is, the transporting unit 3 has a circulation transport path configured by the first transverse unit 31, buffer unit 33, second transverse unit 32, rack feeder 35, and third transverse unit 36. Thus, the transporting unit 3 can deposit the sample container 100 at the urine sample obtaining position of the urine formed component measurement unit 22 and the urine sample obtaining position of the biochemical measurement unit 23 while the rack 101 is circulating along the circulation transport path.

The transporting unit 3 is provided with barcode readers 37 and 38 to read the barcode (not shown in the drawings) adhered to each sample container 100. Specifically, the barcode reader 37 is disposed along the first transverse unit 31 on the rack feeder 34 side (side in the arrow X2 direction) from the urine sample obtaining position of the urine formed component measurement unit 22. Thus, the barcode reader 37 can read the barcode of the sample container 100 transported by the first transverse unit 31 before the sample is aspirated by the aspirating pipette 221. The barcode reader 38 is disposed along the second transverse unit 32 on the buffer 33 side (side in the arrow X2 direction) from the urine sample obtaining position of the biochemical measurement unit 23, Thus, the barcode reader 38 can read the barcode of the sample container 100 transported by the second transverse unit 32 before the sample is aspirated by the aspirating pipette 231. The sample ID (barcode information) read by the barcode readers 37 and 38 is transmitted by the CPU 25a of the controller 25 through the communication unit 24 to the needed data analysis unit 4. Note that the barcode adhered to each sample container 100 is unique for each individual urine sample and is used to manage the urine samples.

The data analysis unit 4 is a computer mainly configured by a controller 41 (refer to FIG. 4), display unit 42, and input device 43, as shown in FIGS. 1 and 4.

As shown in FIG. 4, the controller 41 is mainly configured by a CPU 411, ROM 412, RAM 413, hard disk 414, reading device 415, I/O interface 416, communication interface 417, and image output interface 418. The CPU 411, ROM 412, RAM 413, hard disk 414, reading device 415, I/O interface 416, communication interface 417, and image output interface 418 are mutually connected via a bus 419.

The CPU 411 is capable of executing computer programs stored in the ROM 412 and computer programs loaded in the RAM 413. The computer functions as the data analysis unit 4 when the CPU 411 executes the urine sample analysis program 44a and diagnostic support program 44b which will be described later.

] In the present embodiment, the CPU 411 of the data analysis unit 4 analyzes the measurement data of the urine formed component measurement unit 22 received through the communication unit 417, and obtains a urine formed component analysis result. Specifically, the CPU 411 is configured to obtain red blood cell distribution information as red blood cell morphological information, and cell concentration information that represents the number of each kind of cell by analyzing the urine formed component measurement data of red blood cells (RBC), white blood cells (WBC), epithelial cells (EC), casts (CAST), and bacteria (BACT). The CPU 411 is further configured to obtain quantitative biochemical analysis results by analyzing the measurement data obtained by the biochemical measurement unit 23 and received through the communication unit 417. Specifically, the CPU 411 is configured to obtain the micro total protein concentration representing the concentration of the total protein (micro total protein) in the urine sample, micro albumin concentration representing the albumin concentration (micro albumin) in the urine sample, micro cholesterol concentration representing the cholesterol concentration (micro cholesterol) in the urine sample, and micro creatinine concentration representing the creatinine concentration (micro creatinine) in the urine sample. The CPU 411 jointly analyzes both the measurement data obtained by the urine formed component measurement unit 22 and the measurement data obtained by the biochemical measurement unit 23.

The CPU 411 is configured to obtain the estimated excretion values per day of micro total protein, micro albumin, and micro cholesterol by performing creatinine correction using the obtained creatinine concentration.

Specifically, the CPU 411 obtains the micro total protein estimated excretion value (m-TP) per day, micro albumin estimated excretion value (m-ALB) per day, and micro cholesterol estimated excretion value (m-CHO) per day based on equation (1) below.

$$Z = X \times a \div Y \cdots\cdots (1)$$

In equation (1), Z represents the estimated excretion value (mg) per day of the target component, X represents the concentration (mg/dL) of the target component, Y represents the micro creatinine concentration (g/dL), and a represents a coefficient derived from the body type, age, and sex of the subject. Note that in equation (1), the amount of creatinine excretion in urine per day by a subject with standard muscle mass is assumed to be 1 g. The creatinine concentration is known to be proportional to the muscle mass of the subject, so that it is desirable that a>1 when the subject is, for example, a young male adult with excess muscle mass, and a<1 when the subject is an older female with less muscle mass.

The ROM 412 is configured by a mask ROM, PROM, EPROM, EEPROM or the like, and stores the computer programs to be executed by the CPU 411 as well as the data used by these computer programs.

The RAM 413 is configured by SRAM, DRAM. or the like. The RAM 413 is used when reading the computer programs stored in the ROM 412 and the hard disk 414. The RAM 413 is also used as the work area of the CPU 411 when the CPU 411 executes these computer programs.

The hard disk 414 stores an operating system, application programs and the like, and the various computer programs to be executed by the CPU 411 as well as the data used in the execution of the computer programs. The urine sample analysis program 44a and diagnostic support program 44b are installed on the hard disk 414.

The reading device 415 is configured by a floppy disk drive, CD-ROM drive, DVD-ROM drive or the like, and is capable of reading computer programs or data recorded on a portable recording medium 44. The portable recording medium 444 stores the urine sample analysis program 44a and the diagnostic support program 44b, and the computer can read the urine sample analysis program 44a and the diagnostic support program 44b from the portable recording medium 44 and install the urine sample analysis program 44a and the diagnostic support program 44b on the hard disk 414.

Note that the urine sample analysis program 44a and the diagnostic support program 44b can not only be provided by the portable recording medium 44, the urine sample analysis program 44a and the diagnostic support program 44b may also provided over an electrical communication line from an external device which is connected to the computer via the electrical communication line (either wireless or wired) so as to be capable of communication. For example, the urine sample analysis program 44a and the diagnostic support program 44b may be stored on the hard disk of a server computer on the Internet so that the computer can access the server computer, download the urine sample analysis program 44a and the diagnostic support program 44b, and install the urine sample analysis program 44a and the diagnostic support program 44b on the hard disk 414.

An operating system that provides a graphical user interfaced environment, such as, for example, Microsoft Windows (registered trademark of Microsoft Corporation, USA), may also be installed on the hard disk 414. In the following description, the urine sample analysis program 44a and the diagnostic support program 44b also operate on this operating system.

The I/O interface 416 is configured by a serial interface such as a USB, IEEE1394, RS232C or the like, parallel interface such as SCSI, IDE, IEEE1284 or the like, analog interface such as a D/A converter, A/D converter or the like. The I/O interface 416 is connected to the input device 43 so that a user can input data into the computer using the input device 43. Thus, the user can issue a measurement instruction (input a measurement order) from the data analysis unit 4 to the urine formed component measurement unit 22 and issue measurement instruction (input a measurement order) to the biochemical measurement unit 23.

The communication unit 417 is an Ethernet (registered trademark) interface. The data analysis unit 4 is configured to receive measurement data through the communication unit 417 from the urine formed component measurement unit 22 and biochemical measurement unit 23. The data analysis unit 4 is further configured to receive, through the communication unit 417, urine qualitative analysis result information from the external urine qualitative analyzer 5. The data analysis unit 4 also transmits controls signals through the communication unit 417 to the transport unit 3 and urine sample measurement unit 2.

The image output interface 418 is connected to the display unit42 which is configured by an LCD, CRT or the like, and outputs image signals corresponding to the image data from the CPU 411 to the display unit 42. The display unit 42 displays images (screens) according to the input image signals.

As shown in FIG. 5, the urine qualitative analyzer 5 has controller 51 configured by a CPU, ROM, RAM and the like, and further has a communication unit 52 for sending and receiving data to/from the data analysis unit 4, and first measurement unit 53 and second measurement unit 54 for performing measurements of the urine sample. The urine qualitative analyzer 5 has the function of performing qualitative evaluations of occult blood concentration, protein concentration, white blood cell concentration, nitrite concentration, and glucose concentration.

The controller 51 classifies the occult blood concentration, protein concentration, white blood cell concentration, nitrite concentration, and glucose concentration measured by the first measurement unit 53 (described later) in nine stages, that is, (-), ($\pm$), (+), (2+), (3+), (4+), (5+), (6+), (7+). The controller 51 classifies the urine sample in these none stages based on the degree of color change of a litmus paper used by the first measurement unit 53. The controller 51 sends this classification result (analysis result) information and the specific gravity information obtained by the second measurement unit 54 through the communication unit 52 to the data analysis unit 4.

The first measurement unit 53 is configured to measure the occult blood concentration, protein concentration, white blood cell concentration, nitrite concentration, and glucose concentration of the urine sample. The second measurement unit 54 is configured to measure the specific gravity of the urine sample.

The operation of the urine sample analysis process performed by the urine sample analysis program 44a of the present embodiment of the urine analyzer 1 is described below with reference to FIG. 6.

In step S1, the CPU 411 receives, from the controller 25 through the communication unit 41, the urine sample ID (barcode information) of the measurement target that was read by the barcode reader 37. In step S2, the CPU 411 determines whether there is a measurement instruction (measurement order) for the urine formed component measurement unit 22 from the user, and proceeds to step S6 when there is a measurement instruction. When there is not a measurement instruction, however, the CPU 411 determines in step S3 whether external data have been received, and proceeds to step S9 when not external data have been received. The external data are data obtained from the external urine qualitative analyzer 5. When external data have been received, the CPU 411 determines in step S4 whether a measurement must be performed by the urine formed component measurement unit 22 based on the external data. Specifically, for example, when urine qualitative analysis result information has been received from the urine qualitative analyzer 5, the CPU 411 determines whether the urine formed component measurement unit 22 must perform a measurement based on the degree of occult blood concentration classified in nine stages. The process continues to step S6 if the urine formed component measurement unit 22 must perform a measurement in step S5, whereas the process advances to step S9 if the urine formed component measurement unit 22 is not required to perform a measurement.

In step S6, the CPU 411 issues a measurement instruction to the urine formed component measurement unit 22. Specifically, the controller 25 of the urine sample measurement unit 2 received, through the communication unit 24, a measurement instruction signal sent by the CPU 411 through the communication unit 417, and the controller 25 starts the measurement operation of the urine formed component measurement unit 22. Thus, the urine formed measurement component measurement unit 22 aspirates the urine sample measurement target from the sample container 100 disposed at the urine sample obtaining position, and obtains the measurement data relating to the red blood cells (RBC), white blood cells (WBC), epithelial cells (EC), urinary casts (CAST), and bacteria (BACT) in the urine sample. The controller 52 transmits the urine formed component measurement data obtained by the urine formed component measurement unit 22 to the data analysis unit 4 through the communication unit 24. Thereafter, in step S7, the CPU 411 receives the measurement data relating to the red blood cells (RBC), white blood cells (WBC), epithelial cells (EC), urinary casts (CAST), and bacteria (BACT) in the urine sample from the urine formed component measurement unit 22. In step S8, the CPU 411 then analyzes the received urine formed component measurement data. The red blood cell particle distribution information is then obtained as the morphological information of the red blood cells and the concentration information of each type of cell representing the cell counts of the red blood cells (RBC), white blood cells (WBC), epithelial cells (EC), urinary casts (CAST), and bacteria (BACT) is then obtained as the urine formed component analysis result.

In step S9, the CPU 411 determines whether there is a measurement instruction (measurement order) for the biochemical measurement unit 23 from the user, and proceeds to step S12 when there is a measurement instruction. When there is no measurement instruction, the CPU 411 determines in step S10 whether a measurement is required by the biochemical measurement unit 23 based on the urine formed component analysis result. Specifically, if the red blood cell concentration is below a predetermined threshold value, the CPU 411 determines that measurement is required by the biochemical measurement unit 23, whereas the CPU 411 determines that measurement is not required by the biochemical measurement unit 23 when the red blood cell concentration is equal to or above the predetermined threshold value. In this case, whether a measurement is required by the biochemical measurement unit 23 may be determined based on the concentration information of a cell other than the red blood cells, or whether a measurement is required by the biochemical measurement unit 23 may be determined based on the concentration information of a plurality of types of cells. Whether a measurement is required by the biochemical measurement unit 23 may also be determined based on the red blood cell particle distribution information. The process moves to step S12 when the CPU 411 has determined that measurement is required by the biochemical measurement unit 23 in step S11, whereas the process advances to step S15 when the CPU 411 has determined that measurement by the biochemical measurement unit 23 is unnecessary.

In step S12, the CPU 411 issues a measurement instruction to the biochemical measurement unit 23. Specifically, the controller 25 of the urine sample measurement unit 2 received, through the communication unit 24, a measurement instruction signal sent by the CPU 411 through the communication unit 417, and the controller 25 starts the measurement operation of the biochemical measurement unit 23. Thus, the biochemical measurement unit 23 aspirates the measurement target urine sample from the sample container 100 disposed at the urine sample obtaining position, and obtains the biochemical quantitative measurement data of the total protein, albumin, cholesterol, and creatinine in the urine sample. The controller 52 transmits the biochemical quantitative measurement data obtained by the biochemical measurement unit 23 to the data analysis unit 4 through the communication unit 24. Thereafter, in step S13, the CPU 411 receives the biochemical quantitative measurement data of the total protein, albumin, cholesterol, and creatinine in the urine sample from the biochemical measurement unit 23. In step S14, the CPU 411 analyzes the received biochemical quantitative measurement data. Thus, the micro total protein concentration, micro albumin concentration, micro cholesterol concentration, and micro creatinine concentration are received as biochemical quantitative analysis results. The micro total protein estimated excretion value (m-TP) per day, micro albumin estimated excretion value (m-ALB) per day, and micro cholesterol estimated excretion value (m-CHO) per day are then obtained as biochemical quantitative analysis results by correcting the creatinine value using the micro creatinine concentration.

In step S 15, the CPU 411 determines whether both the urine formed component analysis results and the biochemical quantitative analysis results have been obtained, and advances to step S17 when either one of the two analysis results is missing. When both analysis results have been obtained, the CPU 411 executes the operation of the diagnostic support information obtaining process in step S16.

The of the diagnostic support information obtaining process performed by the diagnostic support program 44b in step S16 of FIG. 6 is described below with reference to FIG. 7.

In step S161, the CPU 411 determines whether the micro total protein estimated excretion value (m-TP) obtained as a biochemical quantitative analysis result is less than the threshold value P, and the process advances to step S162 when the estimated value is less than the threshold value P. In step S162, the CPU 411 determines whether the micro albumin estimated excretion value (m-ALB) obtained as a biochemical quantitative analysis result is less than the threshold value M, and the process advances to step S163 when the estimated value is less than the threshold value M.

In step S163, the CPU 411 determines whether the red blood cell concentration obtained as a urine formed component analysis result is less than a predetermined threshold value L1, and when the RBC concentration is less than the threshold value L1, the CPU 411 determines in step S164 there is no diagnostic support information. That is, the CPU 411 determines that there is no diagnostic support information when all conditions (1) through (3) are satisfied relative to the target urine sample, the conditions being: (1) the micro total protein estimated excretion value (m-TP) is less than the threshold value P, (2) the micro albumin estimated excretion value (m-ALB) is less than the threshold value M1, and (3) the red blood cell concentration is less than the predetermined threshold value L1.

On the other hand, when the red blood cell concentration is equal to or greater than the predetermined threshold value L1 in step S163, the CPU 411 determines in step S165 whether the red blood cells in the urine sample are uniform red blood cells. Specifically, the CPU 411 determines whether the majority of the red blood cells in the urine sample are uniform red blood cells with a low degree of morphological deformation (scant deformation) or the majority of red blood cells are nonuniform red blood cells with a high degree of morphological deformation (nonuniform red blood cells). In the case of uniform red blood cells, the CPU 411 determines in step S166 that there is a possibility of urinary disease, whereas the CPU 411 determines in step S 167 that there is a possibility of renal disease in the case of nonuniform red blood cells. Note that whether red blood cells have been present in the urine over a long period can be estimated by determining whether the red blood cells are uniform, and there is concern of renal disease when red blood cells have been present in the urine over a long period, whereas there is at least a possibility of urinary disease when red blood cells have been present in the urine over a short time.

That is, it is possible to determine there is a possible suspicion of renal disease since there is a high degree of morphological deformation and nonuniform red blood cells when red blood cells have been present in the urine over a long period, and it is possible to determine there is at least a possibility of urinary disease since there is a low degree of morphological deformation and uniform red blood cells when red blood cells have been present in the urine over a short period. Note that it may also be determined that there is a suspicion of renal disease in addition to urinary disease even when red blood cells have not been present in the urine over a short period and there is a low degree of morphological deformation. In the present embodiment, diagnostic support information relating to the suspicion of urinary disease as well as renal disease is obtained, and that the suspicion of renal disease and the suspicion of urinary disease are determined based on the red blood cell morphological information.

When the micro total protein estimated excretion value (m-TP) is equal to or greater than the threshold value P, or when the micro albumin estimated excretion value (m-ALB) is equal to or greater than the threshold value M1, the CPU 411 determines in step S168 whether the micro cholesterol estimated excretion value (m-CHO) obtained as a biochemical quantitative analysis result is less than a threshold value N. When the estimated value is less than the threshold value N, the CPU 411 determines in step S169 whether the red blood cell concentration obtained as a urine formed component analysis result is less than a predetermined threshold value L2, and when the RBC concentration is less than the threshold value L2, the CPU 411 determines in step S170 there is a suspicion of nonprogressive renal disease.

When the red blood cell concentration is equal to or greater than the threshold value L2 in step S169, the CPU 411 determines in step S171 whether the red blood cells in the urine sample are uniform red blood cells, and when the red blood cells are uniform cells, the CPU 411 determines in step S172 that there is a suspicion of urinary disease and a suspicion of renal disease. When the cells are not uniform red blood cells, the CPU 411 determines in step S173 that there is a suspicion of renal disease. Note that the predetermined concentration L1 may be identical to the predetermined concentration L2, although it is preferred that the predetermined concentration L1 is less than the predetermined concentration L2.

When the micro cholesterol estimated excretion value (m-CHO) is equal to or greater than the threshold value N in step S168, the CPU 411 determines in step S174 whether the red blood cell concentration obtained as a urine formed component analysis result is less than the predetermined value L2, and when the RBC concentration is less than the threshold value L2, the CPU 411 determines in step S175 that there is a suspicion of progressive renal disease.

When the red blood cell concentration is equal to or greater than the threshold value L2 in step S174, the CPU 411 determines in step S176 whether the red blood cells in the urine sample are uniform red blood cells, and when the red blood cells are uniform cells, the CPU 411 determines in step S177 that there is a suspicion of urinary disease and a suspicion of renal disease. When the cells are not uniform red blood cells, the CPU 411 determines in step S178 that there is a suspicion of renal disease.

In the embodiment described above, when the micro total protein estimated excretion value (m-TP) is less than the threshold value P or the micro albumin estimated excretion value (n-ALB) is less than the threshold value M1, a determination of suspected renal disease (including strong suspicion) is obtained regardless of the micro cholesterol estimated excretion value (m-CHO) and whether the RBC concentration value indicates uniform red blood cells.

When the micro total protein estimated excretion values (m-TP) is less than the threshold value P or the micro albumin estimated excretion value (m-ALB) is less than the threshold M1, and the micro cholesterol estimated excretion value (m-CHO) is also less than the threshold value N, a suspicion of urinary disease and strong suspicion of renal disease are determined regardless of the red blood cell concentration and whether the RBC are uniform. That is, the degree of renal disease is determined based on the micro cholesterol estimated excretion value (m-CHO). When the micro total protein estimated excretion value (m-TP) is less than the threshold value P or the micro albumin estimated excretion value (m-ALB) is less than the threshold value M1, and the micro cholesterol estimated excretion value (m-CHO) is equal to or greater than the threshold value N, a determination of progressive renal disease is obtained regardless of the red blood cell concentration and whether the RBC are uniform cells. In the present embodiment, progressive renal disease is thus determined based on the micro cholesterol estimated excretion value (m-CHO).

Note that in the present embodiment diagnostic support information can be obtained relating to a suspicion of renal disease indicated by abnormal values in the biochemical quantitative analysis, for example, IgA nephropathy, acute progressive nephritis, diabetes mellitus and the like, by performing a biochemical component quantitative examination in conjunction with a urine formed component examination as described above. Diagnostic support information can also be obtained relating to the suspicion of diseases such as cystitis, urethritis and the like as urinary tract disease not involving the kidneys.

After the diagnostic support information obtaining process of step S16, the CPU 411 displays the analysis result and diagnostic support screen 421 on the display unit 42 in step S17 of FIG. 6, and the operation of the urine sample analysis process ends, as shown in FIG. 8. When re-analysis of the corresponding urine sample is desired, the REVIEW display 421 a is displayed in a red-colored highlight on the analysis result and diagnostic support screen 421. The analysis result and diagnostic support screen 421 also has a qualitative data region 421 b for displaying urine qualitative analysis results received from the external qualitative urine analyzer 5, a formed component analysis results region 421 c for displaying the urine formed component analysis results, and a comment field 421 d. The analysis result and diagnostic support screen 421 further has a quantitative data region 421e for displaying biochemical quantitative, analysis results, diagnostic support information region 421f for displaying diagnostic support information, and subject information region 421g for displaying subject information. Morphological information of the red blood cells in the urine sample is also displayed in the formed component analysis result region 421 c. In FIG. 8, the majority of the red blood cells in the measured urine sample are deformed red blood cells.

The urine qualitative analysis results for both the previous and current results of each component are displayed in the qualitative data region 421b. The formed component analysis region 421c displays the concentration values for both the previous and current results of each cell, that is, red blood cells (RBC), white blood cells (WBC), epithelial cells (EC), casts (CAST), and bacteria (BACT). The formed component analysis region 421 c also displays two-dimensional scattergrams plotting the particles in the urine sample based on the scattered light intensity and fluorescent light intensity.

The quantitative data region 421e displays the concentration values for both the previous and current results of each component, that is, micro total protein concentration (TP), micro albumin concentration (ALB), micro cholesterol concentration (CHO), and micro creatinine concentration (CRE). Note that the quantitative data region 421e can also display the micro total protein estimated excretion value (m-TP) per day, micro albumin estimated excretion value (m-ALB) per day, and micro cholesterol estimated excretion value (m-CHO) per day by changing the display setting. The diagnostic support information region 421f displays both the previous diagnostic support information and the current diagnostic support information obtained by the diagnostic support information obtaining process shown in FIG. 7. The subject information region 421g displays the subject identification information which includes the subject name, birth date and the like.

In the present embodiment described above, the data analysis unit 4 is provided for obtaining concentration information of each cell and red blood cell morphology information by analyzing the measurement data of red blood cells, white blood cells, epithelial cells, casts and bacteria obtained by the urine formed component measurement unit 22, and obtaining micro total protein concentration, micro albumin concentration, micro cholesterol concentration, and micro creatinine concentration in the urine sample by analyzing the biochemical quantitative measurement data obtained by the biochemical measurement unit 23. Thus, the red blood cell morphological information and concentration information of red blood cells, white blood cells, epithelial cells, casts and bacteria are obtained, and micro total protein concentration, micro albumin concentration, micro cholesterol concentration, and micro creatinine concentration are obtained in the same examination stage. Therefore, urine sediment analysis results and urine biochemical analysis results can both be obtained at an early stage, and as a result analysis results useful for diagnosing renal disease can be obtained at an early stage compared to when biochemical analysis (biochemical quantitative measurements) are performed after repeatedly performing urine sediment analysis (urine formed component measurements). Furthermore, analysis means need not be provided separately for the urine formed component measurement unit 22 and biochemical measurement unit 23 since both the measurement data obtained by the urine formed component measurement unit 22 and the biochemical measurement unit 23 are analyzed by a single data analysis unit 4. As a result, the overall structure of the analyzer can be simplified while providing a plurality of measurement units.

In the present embodiment, the operations of both the urine formed component measurement unit 22 and the biochemical measurement unit 23 are controlled by providing a controller 25 for the joint control of operations of the urine formed component measurement unit 22 and the biochemical measurement unit 23. Therefore, the overall structure of the analyzer can be simplified while providing a plurality of measurement units without providing separate controllers for the urine formed component measurement unit 22 and the biochemical measurement unit 23.

In the present embodiment, whether biochemical quantitative measurements are required for total protein, albumin, and cholesterol in a urine sample is determined based on red blood cell concentration information when a user does not issue a measurement instruction to the biochemical measurement unit 23. Thus, the biochemical quantitative measurements can be performed by the biochemical measurement unit 23 when it has been determined that biochemical quantitative measurements by the biochemical measurement unit 23 are required based on the red blood cell concentration even when a user has not issued a measurement instruction for a urine sample to the biochemical measurement unit 23.

In the present embodiment, the creatinine correction is performed using the creatinine concentration for the total protein, albumin, and cholesterol concentrations in the urine sample. Thus, the amounts of components in a urine sample can be more accurately obtained while suppressing variability caused by collection times since estimated values of excretion amounts per day can be obtained for the total protein, albumin, and cholesterol in the urine sample.

In the present embodiment, whether measurements are required by the urine formed component measurement unit 22 is determined based on the urine qualitative analysis result obtained from an externally provided urine qualitative analyzer 5 when a user has not issued a measurement instruction to the urine formed component measurement unit 22. Thus, the measurements can be performed by the urine formed component measurement unit 22 when it has been determined that measurements are required by the urine formed component measurement unit 22 based on the urine qualitative analysis result even when a user has not issued a measurement instruction for a urine sample to the urine formed component measurement unit 22.

Note that, in the embodiments of this disclosure, all aspects are examples and should not be considered as limiting. The scope of the present invention is defined by the scope of the claims and not be the description of the embodiment, and includes all modifications within the scope of the claims and the meanings and equivalences therein.

For example, although the biochemical measurement unit 23 obtains biochemical quantitative measurement data of the total protein, albumin, cholesterol, and creatinine in the urine sample in the above embodiment, the present invention is not limited to this example. A biochemical measurement unit may also be provided that does not obtain measurement data of other components insofar as the biochemical measurement unit is capable of obtaining biochemical quantitative measurement data of at least one component among the total protein, albumin, and cholesterol in the urine sample.

Although the urine formed component measurement unit 22 obtains measurement data relating to the red blood cells, white blood cells, epithelial cells, casts, and bacteria in a urine sample in the above embodiment, the present invention is not limited to this example. A urine formed component measurement unit may also be provided that does not obtain measurement data relating to the red blood cells, white blood cells, epithelial cells, casts, and bacteria in a urine sample insofar as the formed component measurement unit is capable of obtaining measurement data relating to the red blood cells in the urine sample.

Although the data analysis unit 4 obtains red blood cell particle distribution information (morphological information) and concentration information of red blood cells, white blood cells, epithelial cells, casts, and bacteria in the above embodiment, the present invention is not limited to this example. A data analysis unit may also be provided that obtains only one of the red blood cell concentration and red blood cell particle distribution information in a urine sample. In this case, for example, the number of red blood cells may be obtains instead of red blood cell concentration insofar as the numerical information represents the number of red blood cells in the urine sample.

Although the urine formed component measurement unit 22 is configured to measure red blood cells by a flow cytometric method, and the data analysis unit 4 is configured to obtain red blood cell morphological information based on the measurement information obtained by the urine formed component measurement unit 22 in the above embodiment, the present invention is not limited to this example. For example, red blood cell morphological information (information of whether the red blood cells are uniform) may also be obtained by providing a formed state imaging unit for imaging the red blood cells in the urine sample, and subjecting the red blood cell images obtained by the imaging unit to image processing by the data analysis unit 4.

Although the data analysis unit 4 obtains the micro total protein concentration, micro albumin concentration, micro cholesterol concentration and micro creatinine concentration in the above embodiment, the present invention is not limited to this example a data analysis unit may be provided that does not obtain other component concentrations insofar as the data analysis unit obtains at least one of the micro total protein concentration, micro albumin concentration, and micro cholesterol concentration.

Although a single CPU 411 executes the analysis process of the measurement data obtained by the urine formed component measurement unit 22 and the analysis process of the measurement data obtained by the biochemical measurement unit 23 in the above embodiment, CPUs may also be separately provided so as to have one CPU for executing the analysis process of the measurement data obtained by the urine formed component measurement unit 22 and another CPU for executing the analysis process of the measurement data obtained by the biochemical measurement unit 23.

Although a controller 25 jointly controls the operations of both the urine formed component measurement unit 22 and the biochemical measurement unit 23 in the above embodiment, the present invention is not limited to this example. The operations of the urine formed component measurement unit 22 and the biochemical measurement unit 23 may also be controlled by separate controllers. Although the controller 25, which controls the operations of the urine formed component measurement unit 22 and the biochemical measurement unit 23, is provided separately from the data analysis unit 4, the data analysis unit 4 may also control the operations of the urine formed component measurement unit 22 and the biochemical measurement unit 23.

Although the urine formed component measurement unit 22 and the biochemical measurement unit 23 receive measurement instructions from the data analysis unit 4 in the above embodiment, the present invention is not limited to this example. Both the urine formed component measurement unit 22 and the biochemical measurement unit 23 may also perform measurements without receiving a measurement instruction.

Although the data analysis unit 4 determines whether measurement is required by the biochemical measurement unit 23 based on the red blood cell concentration of the urine formed component analysis result when a user does not issue a measurement instruction to the biochemical measurement unit 23 in the above embodiment, the present invention is not limited to this example. The data analysis unit 4 may also determine whether measurement is required by the biochemical measurement unit 23 based on the analysis result of measurement data relating to one of the white blood cells, epithelial cells, casts, and bacteria obtained by the urine formed component measurement unit 22 in addition to the red blood cell concentration of the urine formed component analysis result. The data analysis unit 4 may also determine whether measurement is required by the biochemical measurement unit 23 based on the urine qualitative analysis result received from the urine qualitative analyzer 5. For example, the data analysis unit 4 may also determine whether the protein concentration included in the urine qualitative analysis result received from the urine qualitative analyzer 5 is a level (+) or higher, so as to determine that measurement by the biochemical measurement unit 23 is required when the protein concentration is level (+) or higher.

Although the CPU 411 is configured to determine diseases based on the creatinine-corrected micro total protein estimated excretion value (m-TP), micro albumin estimated excretion value (m-ALB), and micro cholesterol estimated excretion value (m-CHO) in the diagnostic support information obtaining process in the above embodiment, the present invention is not limited to this example. The CPU 411 may also determines the diseases based on the micro total protein concentration, micro albumin concentration, and micro cholesterol concentration before creatinine correction.

Although the urine formed component measurement unit 22 and the biochemical measurement unit 23 are housed in a unitary housing in the above embodiment, the present invention is not limited to this example. The urine formed component measurement unit 22 and the biochemical measurement unit 23 may also be housed in separate housings.

Although aspirating pipettes are separately provided in the urine formed component measurement unit 22 and the biochemical measurement unit 23 in the above embodiment, the present invention is not limited to this example. A single aspirating pipette may be jointly provided for the common use of the urine formed component measurement unit 22 and the biochemical measurement unit 23, so that the urine sample aspirated by the joint aspirating pipette is respectively dispensed to the urine formed component measurement unit 22 and the biochemical measurement unit 23.

Although a urine sample is transported to the urine formed component measurement unit 22 and the biochemical measurement unit 23 as the transporting unit 3 circulates the urine sample between the urine formed component measurement unit 22 and the biochemical measurement unit 23 in the above embodiment, the present invention is not limited to this example. A transporting unit may also be provided that is capable of reciprocating transport of the urine sample in front-to-back directions or side-to-side directions.

Although the data analysis unit 4 determines whether urine formed component measurement is required and whether biological quantitative measurement is required in the above embodiment, a computer other than the data analysis unit 4, such as a host computer or the like, may also perform these determination processes.

Although the urine formed component measurement unit 22 and the biochemical measurement unit 23 are configured to receive into the measuring unit the urine sample that has been transported below the aspirating pipette by the transporting unit via the aspirating pipette in order to perform the measurements, the present invention is not limited to this example. Biochemical measurement may also be performed by using part of a urine sample delivered into a container within the urine formed component measurement unit 22 via the aspirating pipette of the urine formed component measurement unit 22 for the urine formed component measurement, and moving the remaining part of the urine sample in the container to the biochemical measurement unit 23 via a tube or the like to perform the biochemical measurements.

Although the urine sample is received into the urine formed component measurement unit 22 and the biochemical measurement unit 23, respectively, by the aspirating pipettes aspirating the urine sample in a sample container transported to the front of the urine formed component measurement unit 22 and the biochemical measurement unit 23 in the above embodiment, the present invention is not limited to this example. The urine sample may also be received into the urine formed component measurement unit 22 and the biochemical measurement unit 23, respectively, by receiving the sample container transported to the front of the urine formed component measurement unit 22 and the biochemical measurement unit 23 into the housing body, and subsequently the aspirating pipette aspirates the urine sample from the sample container received into the housing body.

Although the data analysis unit 4 determines whether measurements are required by the urine formed component measurement unit 22 based on external data when a measurement instruction (measurement order) has not been issued for the urine formed component measurement unit 22 in the above embodiment, the present invention is not limited to this example. The data analysis unit 4 may determine whether measurement is required by the urine formed component measurement unit 22 based on external data regardless of the presence of a measurement instruction (measurement order) issued to the urine formed component measurement unit 22.

Although the data analysis unit 4 determines whether measurements are required by the biochemical measurement unit 23 based on urine formed component analysis results when a measurement instruction (measurement order) has not been issued for the biochemical measurement unit 23 in the above embodiment, the present invention is not limited to this example. The data analysis unit 4 may determine whether measurement is required by the urine formed component measurement unit 22 based on urine formed component analysis results regardless of the presence of a measurement instruction (measurement order) issued to the biochemical measurement unit 23.

Although the urine formed component analysis results, biochemical quantitative analysis results, and diagnostic support information are displayed in the analysis result and diagnostic support screen 421 on the display unit 42 in the above embodiment, the present invention is not limited to this example. For example, the urine formed component analysis results, biochemical quantitative analysis results, and diagnostic support information may also be printed out via a printer.

## Claims

1. A urine analyzer (1) comprising:

    a transport unit (3) for transporting a sample container containing a urine sample to a first aspirating position and a second aspirating position, wherein the transport unit (3) is configured to transport the sample container to the second aspirating position after being transported to the first aspirating position;
    a first measurement unit (22) for aspirating the urine sample from the sample container at the first aspirating position and for obtaining red blood cell measurement data by performing a measurement of a red blood cell in the aspirated urine sample;
    a second measurement unit (23) for aspirating the urine sample from the sample container at the second aspirating position and for obtaining quantitative measurement data by performing a quantitative measurement of at least one of a protein component and a lipid component in the aspirated urine sample;
    an operation controller (25) shared to control the first measurement unit and the second measurement unit; and
    a data analysis unit (4) for obtaining at least one of numerical information representing the number of red blood cells in the urine sample and morphology information of a red blood cell in the urine sample by analyzing the red blood cell measurement data obtained by the first measurement unit, and for obtaining concentration information representing a concentration of at least one of the protein component and the lipid component in the urine sample by analyzing the quantitative measurement data obtained by the second measurement unit, wherein
    when the data analysis unit (4) has not received an instruction to execute the quantitative measurement from a user, the data analysis unit (4) is adapted to determine a necessity of the quantitative measurement by the second measurement unit based on at least one of the numerical information and morphological information.

2. The urine sample analyzer of claim 1, wherein
the protein component comprises at least one of total protein and albumin; and
the lipid component comprises cholesterol.

3. The urine sample analyzer of claim 1 or 2, wherein the data analysis unit (4) comprises:

    a display (42); and
    a controller (41) for controlling the display so as to display, on a same screen, the concentration information and at least one of the numerical information and morphological information.

4. The urine sample analyzer of claim 3, wherein
the controller (41) is adapted to obtain diagnostic support information relating at least to renal disease based on the concentration information and at least one of the numerical information and morphological information, and controls the display so as to display, on the screen, the obtained diagnostic support information.

5. The urine sample analyzer of any one of claims 1 to 4, wherein
the data analysis unit (4) is adapted to output diagnostic support information relating at least to renal disease based on the concentration information and at least one of the numerical information and red blood cell morphological information.

**6.** The urine sample analyzer of any one of claims 1 to 5, further comprising
a first barcode reader (37) for reading an identification information of the urine sample from the sample container before the urine sample is aspirated by the first measurement unit; and
a second barcode reader (38) for reading the identification information of the urine sample from the sample container before the urine sample is aspirated by the second measurement unit.

**7.** The urine sample analyzer of claim 6, wherein
the transporting unit (3) comprises a circulating transport path on which the first aspirating position and the second aspirating position are arranged, and transports the sample container to the first aspirating position and the second aspirating position along the circulating transport path.

**8.** The urine sample analyzer of any one of claims 1 to 7, wherein
the data analysis unit (4) is adapted to receive an instruction to execute the quantitative measurement from a user; and
the operation controller (25) is adapted to instruct the second measurement unit so as to execute the quantitative measurement when the data analysis unit (4) has received the instruction from the user.

**9.** The urine sample analyzer of any one of claims 1 to 8, wherein
the data analysis unit (4) is adapted to determine that the quantitative measurement by the second measurement unit (23) is necessary when the number of the red blood cells in the urine sample is higher than a threshold value.

**10.** The urine sample analyzer of any one of claims 1 to 9 , wherein
the data analysis unit (4) is adapted to receive an instruction to execute the measurement of red blood cells in the urine sample from a user; and
the operation controller is adapted to instruct the first measurement unit (22) so as to execute the measurement of red blood cells in the urine sample when the data analysis unit (4) has received the instruction to execute the measurement of the red blood cells in the urine sample from the user.

**11.** The urine sample analyzer of any one of claims 1 to 10, wherein
the data analysis unit (4) is adapted to receive a qualitative measurement result relating to the urine sample, and to determine a necessity of the measurement of the red blood cells in the urine sample by the first measurement unit based on the received qualitative analysis result relating to the urine sample.

**12.** The urine sample analyzer of any one of claims 1 to 11, wherein
the second measurement unit (23) is adapted to obtain creatinine quantitative measurement data by further performing quantitative measurement of the creatinine in the urine sample; and
the data analysis unit (4) is adapted to correct the concentration information representing the concentration of at least one of the protein component and lipid component in the urine sample based on the creatinine quantitative measurement data.

**Patentansprüche**

**1.** Urinanalysegerät (1), mit:

einer Transporteinheit (3) zum Transportieren eines Probecontainers, der eine Urinprobe enthält, zu einer ersten Aspirierposition und einer zweiten Aspirierposition, wobei die Transporteinheit (3) konfiguriert ist zum Transportieren des Probencontainers zu der zweiten Aspirierposition, nachdem dieser zu der ersten Aspirierposition transportiert worden ist;
eine erste Messeinheit (22) zum Aspirieren der Urinprobe aus dem Probencontainer an der ersten Aspirierposition, und zum Erhalten von Messdaten roter Blutzellen, indem eine Messung einer roten Blutzelle in der aspirierten Urinprobe durchgeführt wird;
einer zweiten Messeinheit (23) zum Aspirieren der Urinprobe aus dem Probencontainer an der zweiten Aspirierposition, und zum Erhalten von quantitativen Messdaten, indem eine quantitative Messung einer Proteinkomponente und/oder einer Lipidkomponente in der aspirierten Urinprobe durchgeführt wird;
einer Betriebssteuereinheit (25), die gemeinsam die erste Messeinheit und die zweite Messeinheit steuert; und
eine Datenanalyseeinheit (4) zum Erhalten einer numerischen Information zur Darstellung der Anzahl von roten Blutzellen in der Urinprobe und/oder einer Morphologie-Information einer roten Blutzelle in der Urinprobe, durch Analysieren der Messdaten der roten Blutzellen, die durch die erste Messeinheit erhalten werden, und zum Erhalten einer Konzentrationsinformation, die eine Konzentration der Proteinkomponente und/oder der Lipidkomponente in der Urinprobe darstellt, indem die quantitativen Messdaten analysiert werden, die durch die zweite Messeinheit erhalten werden, wobei
wenn die Datenanalyseeinheit (4) von einem Nutzer keine Anweisung empfangen hat, die quantitative Messung auszuführen, die Datenanalyseeinheit (4) ausgelegt ist, eine Notwendigkeit der quantitativen Messung der zweiten Messeinheit zu bestimmen, auf Grundlage der numerischen Information und/oder der morphologischen Information.

**2.** Analysegerät für Urinproben nach Anspruch 1, wobei
die Proteinkomponente Gesamtprotein und/oder Albumin umfasst; und
die Lipidkomponente Cholesterol umfasst.

**3.** Analysegerät für Urinproben nach Anspruch 1 oder 2, wobei die Datenanalyseeinheit (4) umfasst:

eine Anzeige (42); und
eine Steuereinheit (41) zum Steuern der Anzeige, um auf einem gleichen Bildschirm die Konzentrationsinformation sowie die numerische Information und/oder die morphologische Information anzuzeigen.

**4.** Analysegerät für Urinproben nach Anspruch 3, wobei
die Steuereinheit (41) ausgelegt ist zum Erhalten einer diagnostischer Supportinformation in Bezug auf eine Nierenkrankheit auf Grundlage der Konzentrationsinformation und/oder der numerischen Information und/oder der morphologischen Information, und die Anzeige steuert, um auf dem Bildschirm die erhaltene diagnostische Supportinformation anzuzeigen.

**5.** Analysegerät für Urinproben nach irgendeinem der Ansprüche 1 bis 4, wobei
die Datenanalyseeinheit (4) ausgelegt ist zum Ausgeben einer diagnostischen Supportinformation in Bezug auf eine Nierenkrankheit auf Grundlage der Konzentrationsinformation und/oder der numerischen Information und/oder der morphologischen Information der roten Blutzellen.

**6.** Analysegerät für Urinproben nach irgendeinem der Ansprüche 1 bis 5, ferner mit
einer ersten Strichcode-Leseeinheit (37) zum Lesen einer Identifikationsinformation der Urinprobe von dem Probencontainer, bevor die Urinprobe durch die erste Messeinheit aspiriert wird; und
einer zweiten Strichcode-Leseeinheit (38) zum Lesen der Identifikationsinformation der Urinprobe von dem Probencontainer, bevor die Urinprobe durch die zweite Messeinheit aspiriert wird.

**7.** Analysegerät für Urinproben nach Anspruch 6, wobei
die Transporteinheit (3) einen zirkulierenden Transportweg umfasst, auf dem die erste Aspirierposition und die zweite Aspirierposition angeordnet sind, und den Probencontainer zu der ersten Aspirierposition und der zweiten Aspirierposition entlang des zirkulierenden Transportwegs transportiert.

**8.** Analysegerät für Urinproben nach irgendeinem der Ansprüche 1 bis 7, wobei
die Datenanalyseeinheit (4) ausgelegt ist zum Empfangen einer Anweisung von einem Nutzer zum Ausführen der quantitativen Messung; und
die Betriebssteuereinheit (25) ausgelegt ist zum Anweisen der zweiten Messeinheit, die quantitative Messung auszuführen, wenn die Datenanalyseeinheit (4) von dem Nutzer die Anweisung empfangen hat.

**9.** Analysegerät für Urinproben nach irgendeinem der Ansprüche 1 bis 8, wobei
die Datenanalyseeinheit (4) ausgelegt ist zum Bestimmen, dass die quantitative Messung durch die zweite Messeinheit (23) notwendig ist, wenn die Anzahl der roten Blutzellen in der Urinprobe höher als ein Schwellenwert ist.

**10.** Analysegerät für Urinproben nach irgendeinem der Ansprüche 1 bis 9, wobei
die Datenanalyseeinheit (4) ausgelegt ist, von einem Nutzer eine Anweisung zum Ausführen der Messung der roten Blutzellen in der Urinprobe zu empfangen; und
die Betriebssteuereinheit ausgelegt ist, die erste Messeinheit (22) anzuweisen, die Messung der roten Blutzellen in der Urinprobe auszuführen, wenn die Datenanalyseeinheit (4) die Anweisung zum Ausführen der Messung der roten Blutzellen in der Urinprobe von dem Nutzer empfangen hat.

**11.** Analysegerät für Urinproben nach irgendeinem der Ansprüche 1 bis 10, wobei
die Datenanalyseeinheit (4) ausgelegt ist zum Empfangen eines quantitativen Messresultats in Bezug auf die Urinprobe, und zum Bestimmen einer Notwendigkeit der Messung der roten Blutzellen in der Urinprobe durch die erste Messeinheit auf Grundlage des empfangenen qualitativen Analyseresultats bezüglich der Urinprobe.

**12.** Analysegerät für Urinproben nach irgendeinem der Ansprüche 1 bis 11, wobei
die zweite Messeinheit (23) ausgelegt ist zum Erhalten quantitativer Kreatinin-Messdaten, indem ferner eine quantitative Messung von Kreatinin in der Urinprobe durchgeführt wird; und
die Datenanalyseeinheit (4) ausgelegt ist zum Korrigieren der Konzentrationsinformation, die die Konzentration der Proteinkomponente und/oder der Lipidkomponente darstellt, in der Urinprobe, auf Grundlage der quantitativen Kreatinin-Messdaten.

## Revendications

1. Analyseur d'urine (1) comprenant :

   une unité de transport (3) pour transporter un récipient à échantillon contenant un échantillon d'urine jusqu'à une première position d'aspiration et une deuxième position d'aspiration, dans lequel l'unité de transport (3) est configurée pour transporter le récipient à échantillon jusqu'à la deuxième position d'aspiration après l'avoir transporté jusqu'à la première position d'aspiration ;
   une première unité de mesure (22) pour aspirer l'échantillon d'urine hors du récipient à échantillon à la première position d'aspiration et pour obtenir des données de mesure de globules rouges en effectuant une mesure d'un globule rouge dans l'échantillon d'urine aspiré ;
   une deuxième unité de mesure (23) pour aspirer l'échantillon d'urine hors du récipient à échantillon à la deuxième position d'aspiration et pour obtenir des données de mesure quantitative en effectuant une mesure quantitative d'au moins un d'un composant protéique et d'un composant lipidique dans l'échantillon d'urine aspiré ;
   un contrôleur de fonctionnement (25) partagé pour contrôler la première unité de mesure et la deuxième unité de mesure ; et
   une unité d'analyse de données (4) pour obtenir au moins une d'une information numérique représentant le nombre de globules rouges dans l'échantillon d'urine et d'une information de morphologie d'un globule rouge dans l'échantillon d'urine en analysant les données de mesure de globules rouges obtenues par la première unité de mesure, et pour obtenir une information de concentration représentant une concentration d'au moins un du composant protéique et du composant lipidique dans l'échantillon d'urine en analysant les données de mesure quantitative obtenues par la deuxième unité de mesure, dans lequel
   lorsque l'unité d'analyse de données (4) n'a pas reçu d'instruction pour exécuter la mesure quantitative d'un utilisateur, l'unité d'analyse de données (4) est adaptée à déterminer une nécessité de la mesure quantitative par la deuxième unité de mesure sur la base d'au moins une de l'information numérique et de l'information morphologique.

2. Analyseur d'échantillon d'urine selon la revendication 1, dans lequel
   le composant protéique comprend au moins une des protéines totales et de l'albumine totale ; et
   le composant lipidique comprend le cholestérol.

3. Analyseur d'échantillon d'urine selon la revendication 1 ou 2, dans lequel l'unité d'analyse de données (4) comprend :

   un afficheur (42) ; et
   un contrôleur (41) pour contrôler l'afficheur de manière à afficher, sur un même écran, l'information de concentration et au moins une de l'information numérique et de l'information morphologique.

4. Analyseur d'échantillon d'urine selon la revendication 3, dans lequel
   le contrôleur (41) est adapté à obtenir une information de support de diagnostic se rapportant au moins à une maladie rénale sur la base de l'information de concentration et d'au moins une de l'information numérique et de l'information morphologique, et contrôle l'afficheur de manière à afficher, sur l'écran, l'information de support de diagnostic obtenue.

5. Analyseur d'échantillon d'urine selon l'une quelconque des revendications 1 à 4, dans lequel
   l'unité d'analyse de données (4) est adaptée à délivrer en sortie une information de support de diagnostic se rapportant au moins à une maladie rénale sur la base de l'information de concentration et d'au moins une de l'information numérique et de l'information morphologique des globules rouges.

6. Analyseur d'échantillon d'urine selon l'une quelconque des revendications 1 à 5, comprenant en outre un premier lecteur de code à barres (37) pour lire une information d'identification de l'échantillon d'urine provenant du récipient à échantillon avant que l'échantillon d'urine ne soit aspiré par la première unité de mesure ; et
   un deuxième lecteur de code à barres (38) pour lire l'information d'identification de l'échantillon d'urine provenant du récipient à échantillon avant que l'échantillon d'urine ne soit aspiré par la deuxième unité de mesure.

7. Analyseur d'échantillon d'urine selon la revendication 6, dans lequel
   l'unité de transport (3) comprend un chemin de transport de circulation sur lequel la première position d'aspiration et la deuxième position d'aspiration sont agencées, et transporte le récipient à échantillon jusqu'à la première position d'aspiration et la deuxième position d'aspiration le long du chemin de transport de circulation.

8. Analyseur d'échantillon d'urine selon l'une quelconque des revendications 1 à 7, dans lequel
   l'unité d'analyse de données (4) est adaptée à recevoir une instruction d'exécution de la mesure quantitative d'un utilisateur ; et

le contrôleur de fonctionnement (25) est adapté à indiquer à la deuxième unité de mesure d'exécuter la mesure quantitative lorsque l'unité d'analyse de données (4) a reçu l'instruction de l'utilisateur.

**9.** Analyseur d'échantillon d'urine selon l'une quelconque des revendications 1 à 8, dans lequel
l'unité d'analyse de données (4) est adaptée à déterminer que la mesure quantitative par la deuxième unité de mesure (23) est nécessaire lorsque le nombre des globules rouges dans l'échantillon d'urine est supérieur à une valeur de seuil.

**10.** Analyseur d'échantillon d'urine selon l'une quelconque des revendications 1 à 9, dans lequel
l'unité d'analyse de données (4) est adaptée à recevoir une instruction d'exécution de la mesure des globules rouges dans l'échantillon d'urine d'un utilisateur ; et
le contrôleur de fonctionnement est adapté à indiquer à la première unité de mesure (22) d'exécuter la mesure des globules rouges dans l'échantillon d'urine lorsque l'unité d'analyse de données (4) a reçu l'instruction d'exécuter la mesure des globules rouges dans l'échantillon d'urine de l'utilisateur.

**11.** Analyseur d'échantillon d'urine selon l'une quelconque des revendications 1 à 10, dans lequel
l'unité d'analyse de données (4) est adaptée à recevoir un résultat de mesure qualitative se rapportant à l'échantillon d'urine, et à déterminer une nécessité de la mesure des globules rouges dans l'échantillon d'urine par la première unité de mesure sur la base du résultat d'analyse qualitative reçu se rapportant à l'échantillon d'urine.

**12.** Analyseur d'échantillon d'urine selon l'une quelconque des revendications 1 à 11, dans lequel
la deuxième unité de mesure (23) est adaptée à obtenir des données de mesure quantitative de créatinine en effectuant en outre une mesure quantitative de la créatinine dans l'échantillon d'urine ; et
l'unité d'analyse de données (4) est adaptée à corriger l'information de concentration représentant la concentration d'au moins un du composant protéique et du composant lipidique dans l'échantillon d'urine sur la base des données de mesure quantitative de créatinine.

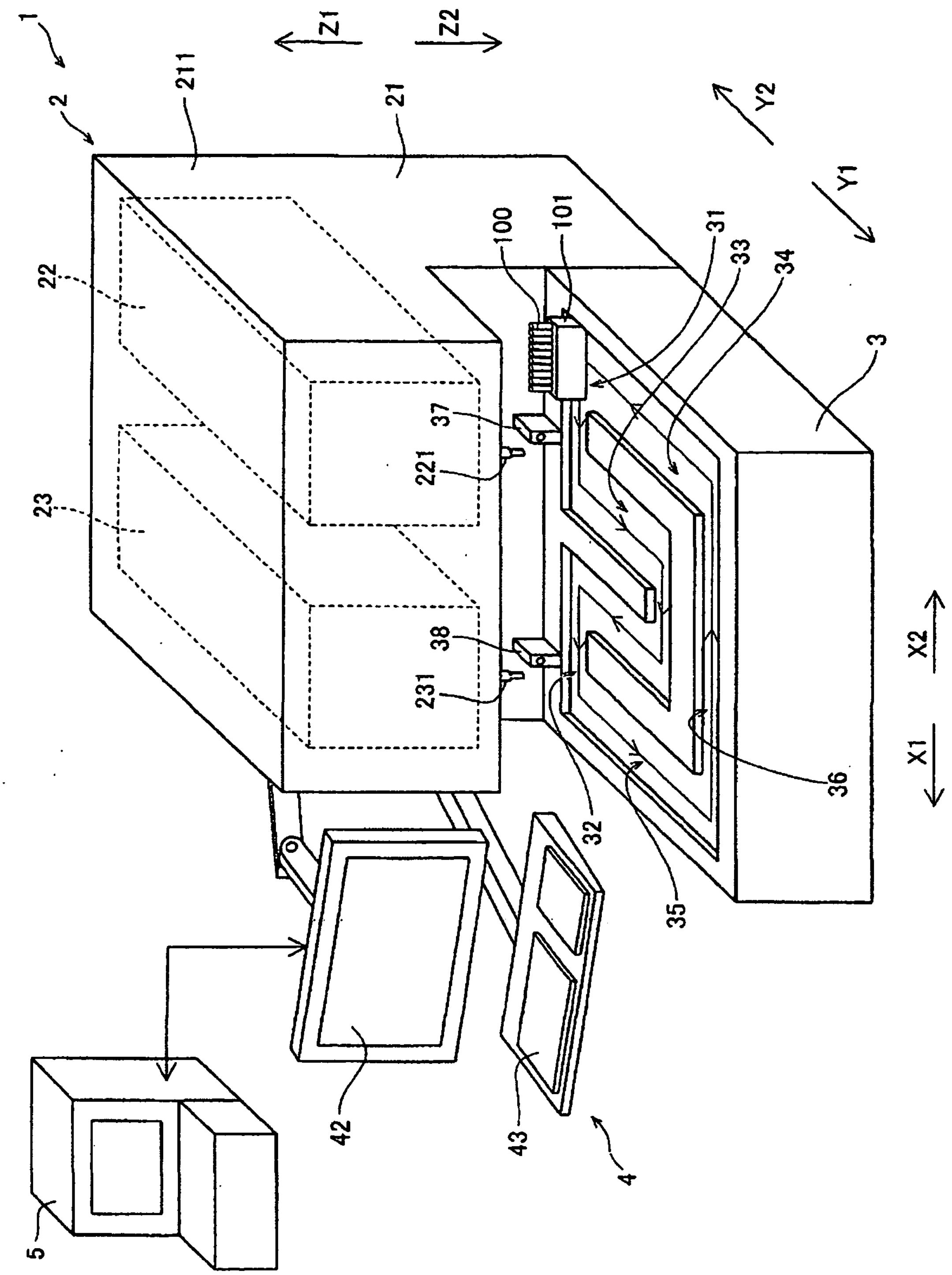
1
2
211
21
Z1
Z2
Y1
Y2
X1
X2
22
23
100
101
31
33
34
3
37
221
38
231
32
35
36
42
43
4
5

Fig. 1

Fig. 2

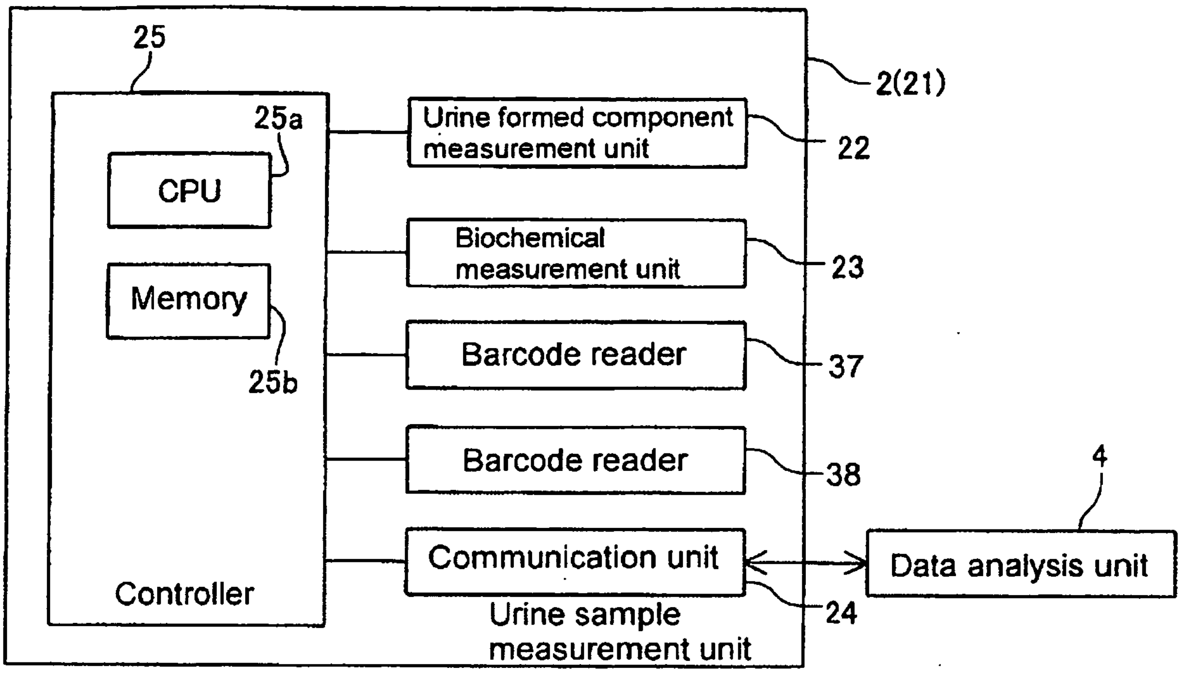
25
25a
CPU
Memory
25b
Controller
Urine formed component measurement unit
Biochemical measurement unit
Barcode reader
Barcode reader
Communication unit
Urine sample measurement unit
2(21)
22
23
37
38
24
4
Data analysis unit

Fig. 3

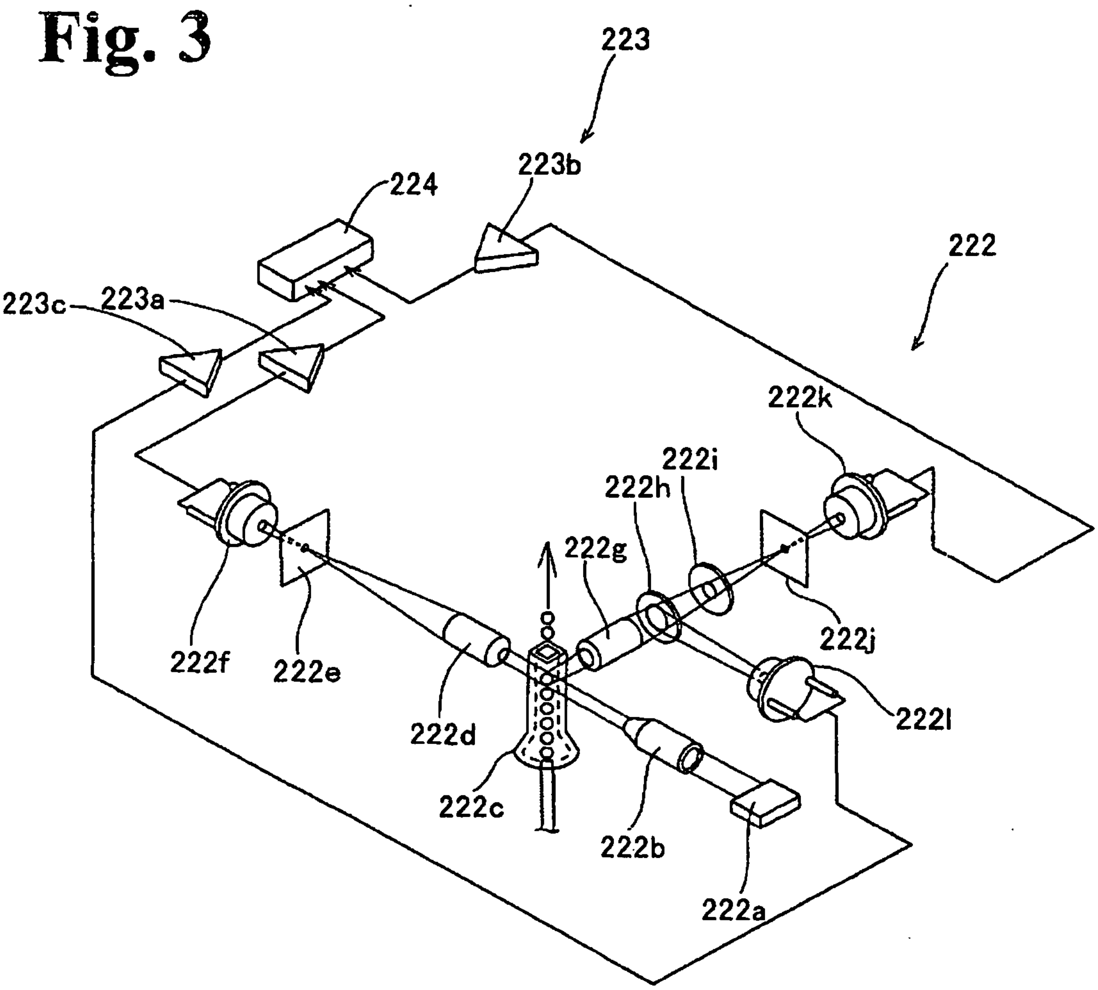
223
224
223b
222
223c
223a
222k
222i
222h
222g
222f
222e
222j
222d
222l
222c
222b
222a

Fig. 4

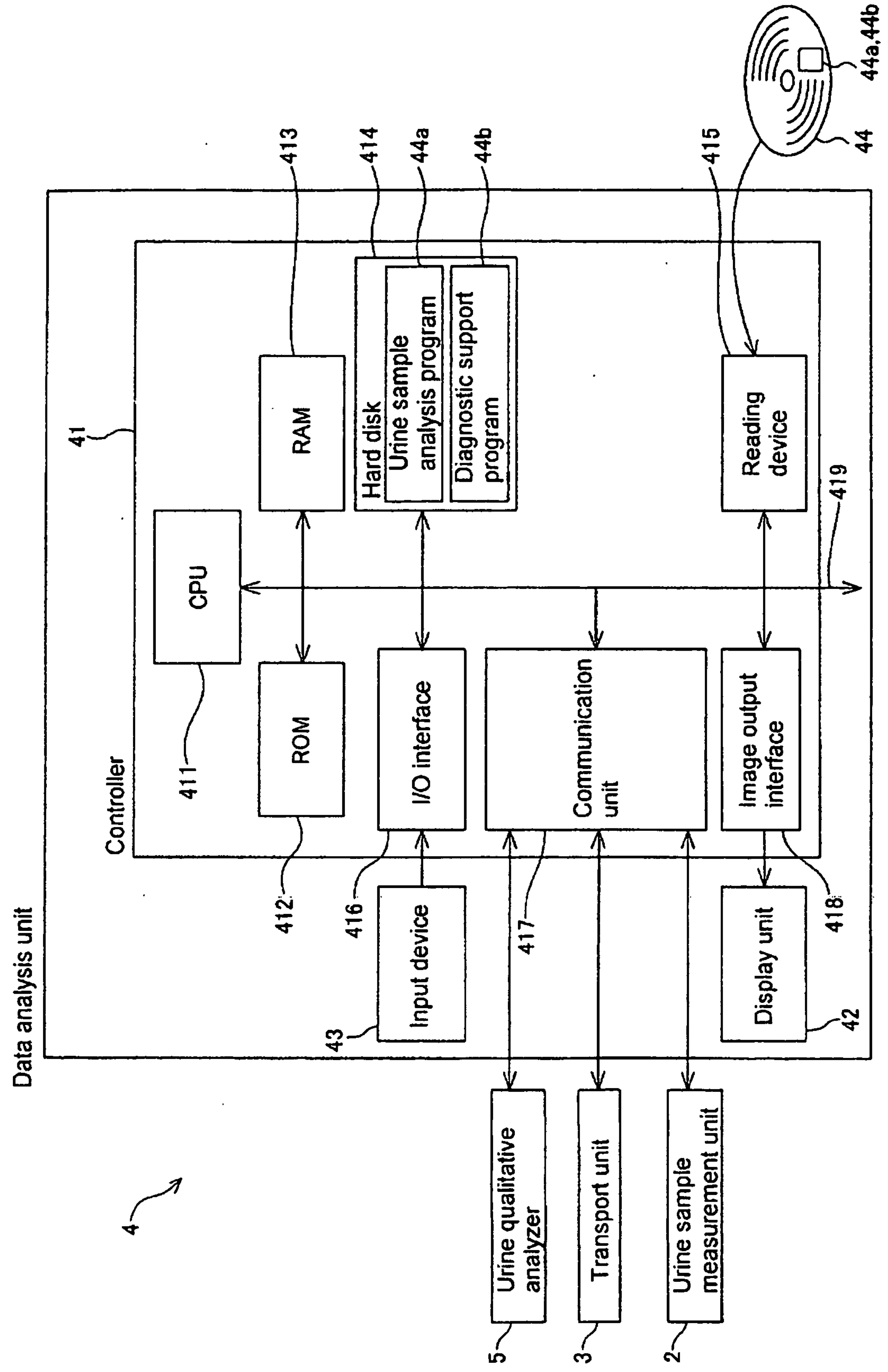
4
Data analysis unit
Controller
CPU
RAM
ROM
Hard disk
Urine sample analysis program
Diagnostic support program
Reading device
I/O interface
Communication unit
Image output interface
Input device
Display unit
Urine qualitative analyzer
Transport unit
Urine sample measurement unit
41
411
412
413
414
44a
44b
415
416
417
418
419
42
43
44
44a,44b
2
3
5

# Fig. 5

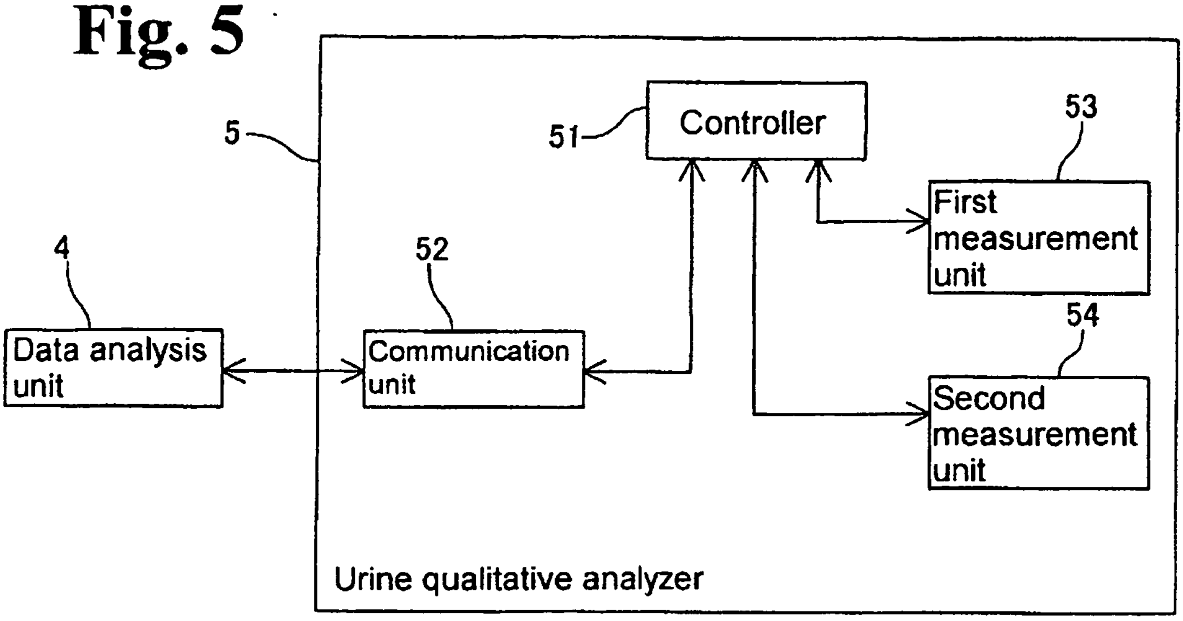
5
4
Data analysis unit
51
Controller
52
Communication unit
53
First measurement unit
54
Second measurement unit
Urine qualitative analyzer

# Fig. 6

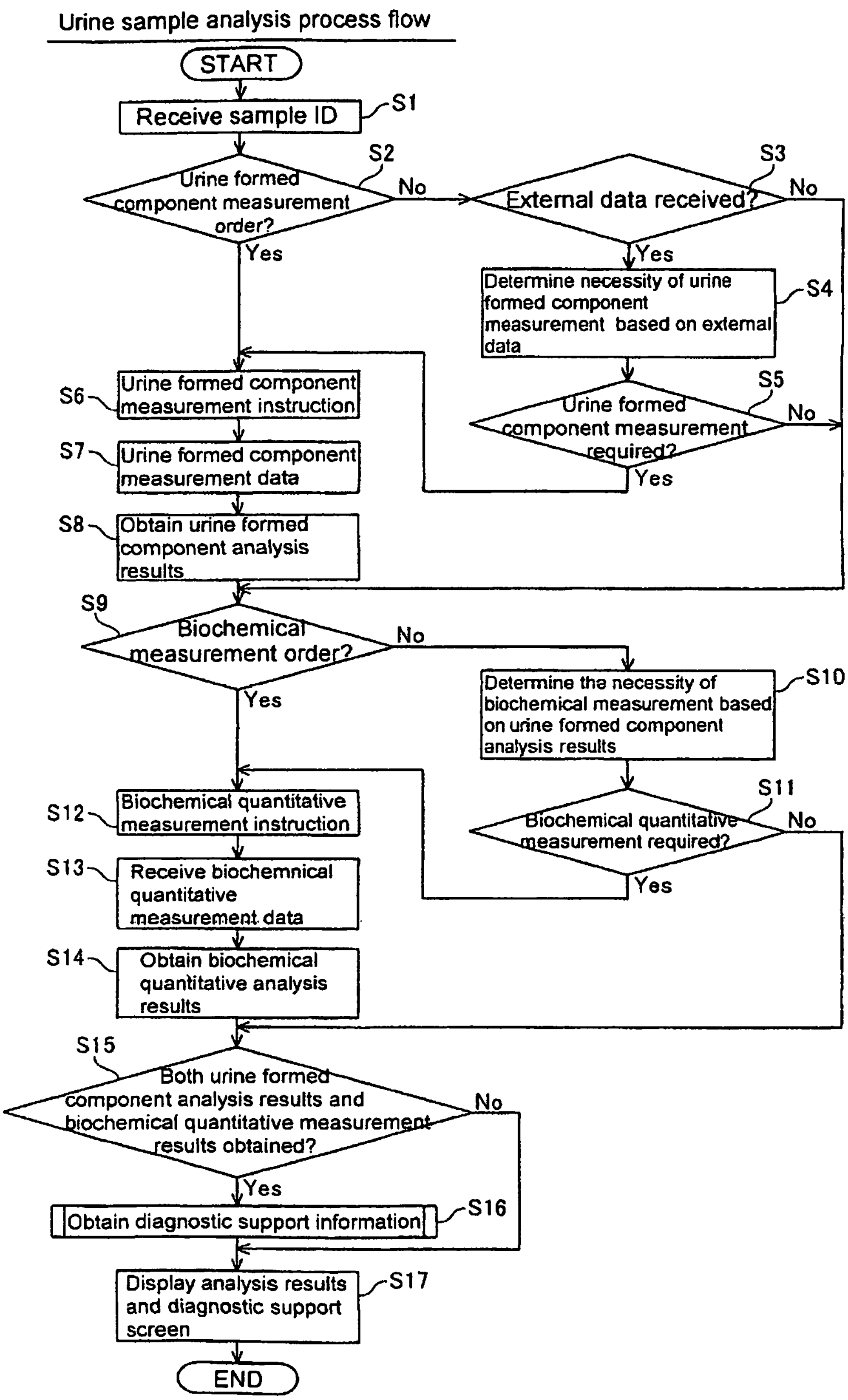
Urine sample analysis process flow
START
Receive sample ID
S1
S2
Urine formed component measurement order?
No
Yes
S3
External data received?
No
Yes
Determine necessity of urine formed component measurement based on external data
S4
S5
Urine formed component measurement required?
No
Yes
S6
Urine formed component measurement instruction
S7
Urine formed component measurement data
S8
Obtain urine formed component analysis results
S9
Biochemical measurement order?
No
Yes
Determine the necessity of biochemical measurement based on urine formed component analysis results
S10
S11
Biochemical quantitative measurement required?
No
Yes
S12
Biochemical quantitative measurement instruction
S13
Receive biochemnical quantitative measurement data
S14
Obtain biochemical quantitative analysis results
S15
Both urine formed component analysis results and biochemical quantitative measurement results obtained?
No
Yes
Obtain diagnostic support information
S16
Display analysis results and diagnostic support screen
S17
END

# Fig. 7

Diagnostic support information obtaining process flow

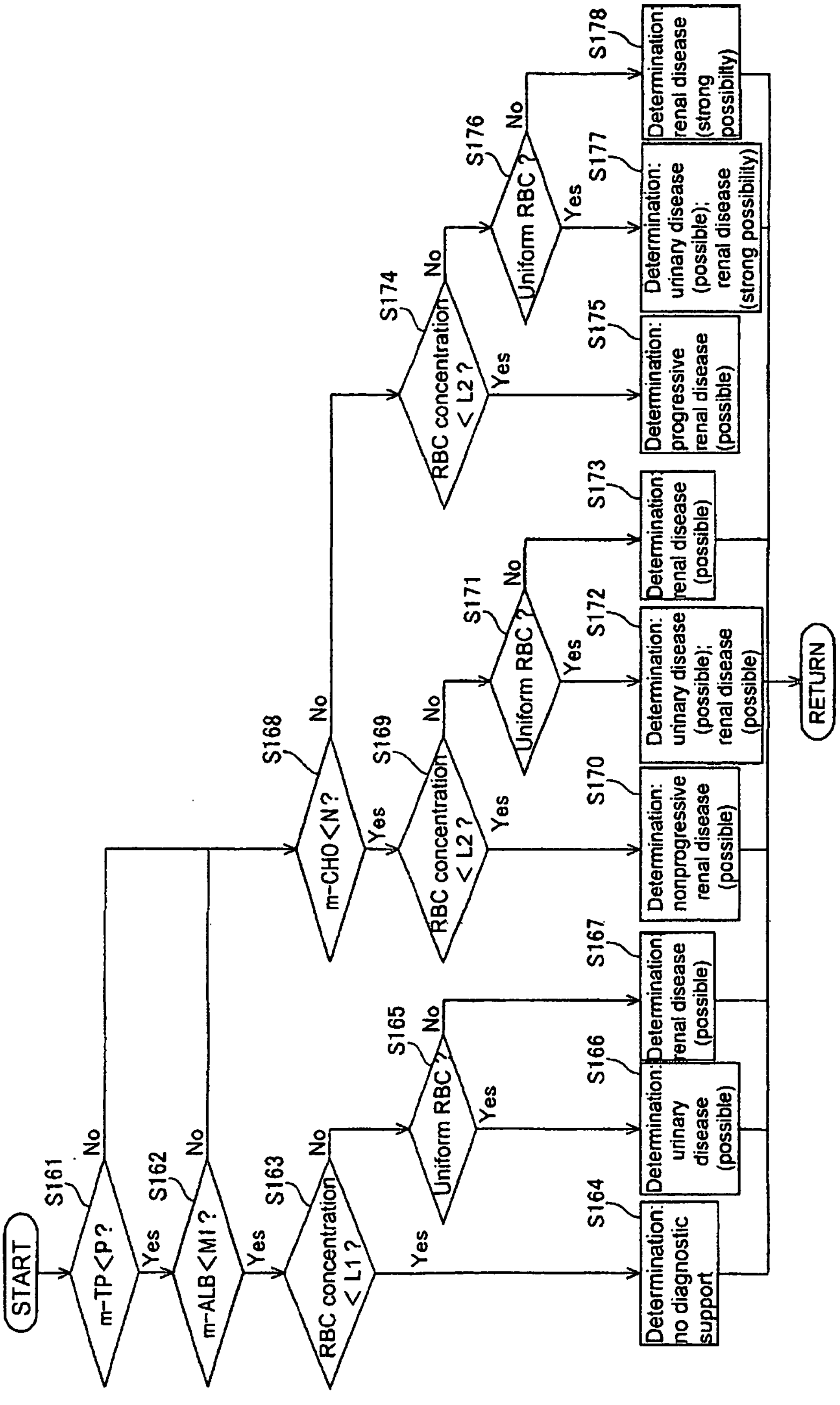

Fig. 8

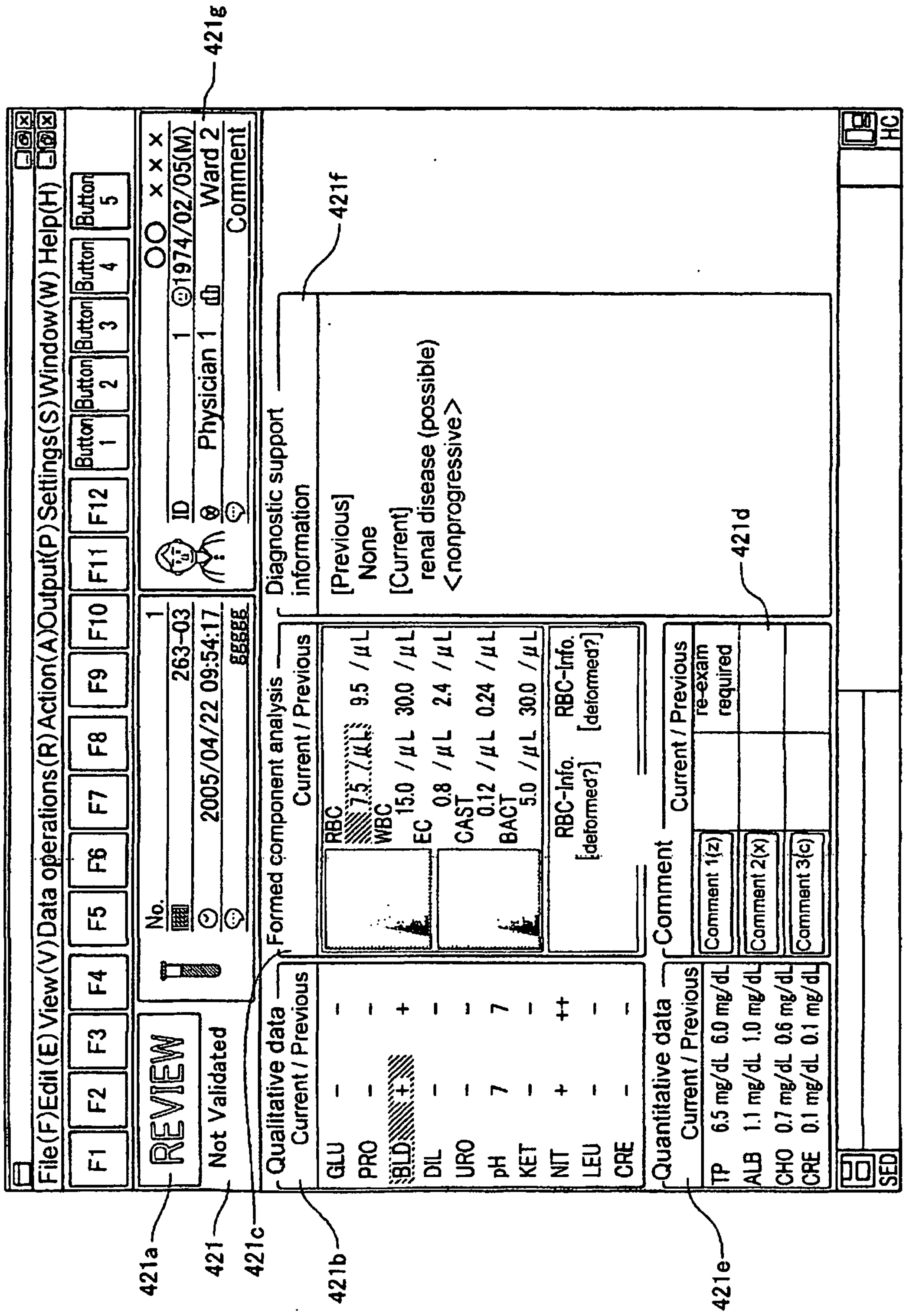
File(F) Edit(E) View(V) Data operations(R) Action(A) Output(P) Settings(S) Window(W) Help(H)
F1 F2 F3 F4 F5 F6 F7 F8 F9 F10 F11 F12
Button 1 Button 2 Button 3 Button 4 Button 5
REVIEW
Not Validated
No. 1
263-03
2005/04/22 09:54:17
ggggg
ID 1
○○ ×××
1974/02/05(M)
Physician 1
Ward 2
Comment
Qualitative data
Current / Previous
GLU − −
PRO − −
BLD + +
DIL − −
URO − −
pH 7 7
KET − −
NIT + ++
LEU − −
CRE − −
Formed component analysis
Current / Previous
RBC 7.5 /μL 9.5 /μL
WBC 15.0 /μL 30.0 /μL
EC 0.8 /μL 2.4 /μL
CAST 0.12 /μL 0.24 /μL
BACT 5.0 /μL 30.0 /μL
RBC-Info. [deformed?]
RBC-Info. [deformed?]
Diagnostic support information
[Previous]
None
[Current]
renal disease (possible)
<nonprogressive>
Quantitative data
Current / Previous
TP 6.5 mg/dL 6.0 mg/dL
ALB 1.1 mg/dL 1.0 mg/dL
CHO 0.7 mg/dL 0.6 mg/dL
CRE 0.1 mg/dL 0.1 mg/dL
Comment
Comment 1(z)
Comment 2(x)
Comment 3(c)
Current / Previous
re-exam required
SED
HC
421
421a
421b
421c
421d
421e
421f
421g

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5325168 A
- JP 2001228158 A
- EP 1857804 A2
- JP 11064218 A